# EUROPEAN PATENT APPLICATION

(11) **EP 3 944 808 A1**
(43) Date of publication of application: **02.02.2022**
(21) Application number: 21188389.7
(22) Date of filing: 29.07.2021
(51) Int. Cl.: A61B 5/053, A61B 5/287, A61B 18/14, G16H 50/20, A61B 5/00, A61B 18/00

(54) **AUTOMATIC SEGMENTATION OF ANATOMICAL STRUCTURES OF WIDE AREA CIRCUMFERENTIAL ABLATION POINTS**

(30) Priority: 30.07.2020 US 202063059060 P; 21.07.2021 US 202117382007
(71) Applicant: Biosense Webster (Israel) Ltd., 2066717 Yokneam (IL)
(72) Inventor: GOLDBERG, Stanislav, Yorkneam (IL); AMIT, Matityahu, Yorkneam (IL); AMOS, Yariv Avraham, Yorkneam (IL)
(74) Representative: Carpmaels & Ransford LLP

(57) **Abstract**

A method and apparatus for implementing an evaluation engine implemented using a processor coupled to a memory. The evaluation engine receives effective points respective to cardiac tissue of a patient. The evaluation engine determines an anatomical structural classification for each of the effective points based on a structural segmentation for the cardiac tissue and provides the anatomical structural classification with the each of the plurality of effective points to support treatment of the cardiac tissue.

## Description

### CROSS REFERENCE TO RELATED APPLICATIONS

This application claims the benefit of U.S. Provisional Patent Application No. 63/059,060, filed July 30, 2020, which is incorporated by reference as if fully set forth.

### FIELD OF INVENTION

The present invention is related to an artificial intelligence and machine learning methods and systems. More particularly, the present invention relates to a system and method utilizing a machine learning algorithm that executes automatic segmentation of anatomical structures of wide area circumferential ablation (WACA) points.

### BACKGROUND

Treatments for cardiac conditions often require heart imaging (i.e., imaging cardiac tissue, chambers, veins, arteries and/or pathways, which is also known as cardiac scanning or cardiac imaging) and subsequent ablation (i.e., removal or destruction of the imaged cardiac tissue, chambers, veins, arteries and/or pathways). In an example, the cardiac condition of atrial fibrillation can be imaged and treated using atrial fibrillation ablation. A specific type of atrial fibrillation ablation is wide area circumferential ablation (WACA).

Conventionally, during the heart mapping phase, WACA achieves right and left pulmonary vein isolation, point by point (e.g., WACA ablation points), to make a circular ring around the left and right pulmonary veins (e.g., in some cases, WACA can extend to a roof of an atria and into a right atria). Yet, WACA is limited by an existence of potential gaps in between the WACA ablation points, an existence of potential gaps in resulting ablation lines, and a dependence by WACA on anatomical structures. For instance, if left untreated during the ablation phase, these gaps could lead to pulmonary vein reconnection and recurrent arrhythmia. To overcome these limitations, conventional methods rely on manual ablation site anatomical segmentation and manual WACA ablation point contiguity estimation by medical professionals. Due to these limitations and in view of conventional manual review methods, a need exists to provide improved methods for predicting potential gaps, providing anatomical segmentation, and providing contiguity estimation for the WACA ablation points.

### SUMMARY

A method and apparatus for implementing an evaluation engine implemented using a processor coupled to a memory are provided. The evaluation engine receives effective points respective to cardiac tissue of a patient. The evaluation engine determines an anatomical structural classification for each of the effective points based on a structural segmentation for the cardiac tissue and provides the anatomical structural classification with the each of the plurality of effective pointsto support treatment of the cardiac tissue.

### BRIEF DESCRIPTION OF THE DRAWINGS

A more detailed understanding may be had from the following description, given by way of example in conjunction with the accompanying drawings, wherein like reference numerals in the figures indicate like elements, and wherein:
FIG. 1 illustrates a diagram of an exemplary system in which one or more features can be implemented;
FIG. 2 illustrates a block diagram of an example system for remotely monitoring and communicating patient biometrics;
FIG. 3 illustrates a graphical depiction of an artificial intelligence system;
FIG. 4 illustrates a block diagram of a method performed in the artificial intelligence system of FIG. 3;
FIG. 5 illustrates a block diagram of a method according to one or more embodiments;
FIG. 6A illustrates an example of an autoencoder structure;
FIG. 6B illustrates a block diagram of a method performed in the autoencoder of FIG. 6A;
FIG. 7 illustrates a block diagram of a method according to one or more embodiments;
FIG. 8 illustrates a graphical image of an example of a graphical user interface providing an image of the PVs with two separate circular rings;
FIG. 9A illustrates a graphical image of a user interface showing datasets;
FIG. 9B illustrates a graphical image of a user interface showing datasets;
FIG. 10 illustrates a graphical depiction of classifying left and right WACA into anatomical structures;
FIG. 11A illustrates a diagram of a right anatomical structure;
FIG. 11B illustrates a diagram of a left anatomical structure;
FIG. 12A illustrates a block diagram of a classification method for an example operation of a random forest for acute reconnection classifier;
FIG. 12B illustrates a block diagram of a classification method for leveraging random forest classification, fully connected dense layers, and a CNN architecture;
FIG. 13 illustrates an exemplary random forest classifier;
FIG. 14A illustrates a block diagram of a classification method for an example operation of a random forest for acute reconnection classifier;
FIG. 14B illustrates a block diagram of a classification method for an example operation of a deep learning method for a redo/acute reconnection classifier; and
FIG. 14C illustrates a graphical image depicting acute reconnection points.

### DETAILED DESCRIPTION

Disclosed herein is a system and method for automatic segmentation of anatomical structures of wide area circumferential ablation (WACA) points. The system and method include artificial intelligence and machine learning. More particularly, this disclosure relates to a system and method for automatic segmentation of anatomical structures of WACA points that includes a machine learning algorithm that executes automatic segmentation of anatomical structures of effective WACA ablation and effective ablation points (e.g., effective points) during the heart mapping phase. For example, the system and method includes a processor executable code or software that is in process operations by, and in processing hardware of, medical device equipment to perform automatic segmentation of anatomical structures of the effective points using random forest regression, fully connected dense layers, and a convolutional neural network (CNN) architecture.

According to an embodiment, the system and method include an evaluation engine that provides a specific multi-step data manipulation of the effective points for automatic anatomical segmentation, contiguity estimation, and gap prediction. For example, during the heart mapping phase, the evaluation engine may determine whether an ablation point is part of a right WACA or a left WACA (e.g., whether each WACA point is within a left circular ring around the left pulmonary vein (PV) or a right circular ring around the right PV) and classifies the left and right WACA points into a number, such as nine, of anatomical structures of for the PVs (e.g., associates each ablation point to one of the anatomical structures). During the heart mapping phase, the evaluation engine may determine whether each of the effective points is good or correct, such that the point helps a medical professional isolate the PVs. In determining good or correct effective points, the evaluation engine may use the right and left WACA information and anatomical structural classification to predict acute reconnections and redo-locations.

For example, pulmonary vein isolation (PVI) is performed with radiofrequency energy in a point-by-point WACA pattern (e.g., a WACA procedure) using an ablation catheter, with an end point of the WACA procedure being a complete pulmonary vein isolation. After a last ablation WACA point, a physician places a mapping catheter sequentially in each of the PVs to determine if spontaneous PV reconnection has occurred. If pulmonary vein reconnection had not occurred, a bolus of intravenous adenosine is administered to unmask any sites of dormant conduction. An ablation may be performed at any sites of reconnection to achieve PVI. The evaluation engine may seek to inform the physician in advance after performing the WACA procedure of the potential reconnection sites, so that ablating those sites may yield PVI. The evaluation engine reduces the need for additional ablations in the second and third phase (e.g., after adenosine administration). Even if there is isolation of the PVs, atrial fibrillation may be repeated after a patient is released from a first WACA session via follow-up ablation sessions. The evaluation engine may provide the physician to re-ablate certain areas (i.e., predict redo locations) during the first WACA session.

The technical effects and benefits of the evaluation engine include automatic anatomical segmentation, contiguity estimation, and potential long term (redo) reconnection sites estimation for ablation points to provide improved image data of cardiac tissue. The improved image data reduces and/or eliminates the potential existence of gaps in between the WACA ablation points and resulting ablation lines and the dependence by WACA on anatomical structures. Thus, the improved image data may be used to effectively treat various cardiovascular diseases by reducing or eliminating the potential for these gaps to lead to PV reconnection and recurrent arrhythmia. The evaluation engine may be practically applied, but not limited to, ablation-ultrasound technologies (e.g., atrial fibrillation ablation and WACA), planning and diagnosis of lesions, and assessment and diagnosis of magnetic resonance to address one or more disease states, such as atrial fibrillation, atrial flutter, general electrophysiology, arrhythmias, ventricular fibrillation, and ventricular tachycardia.

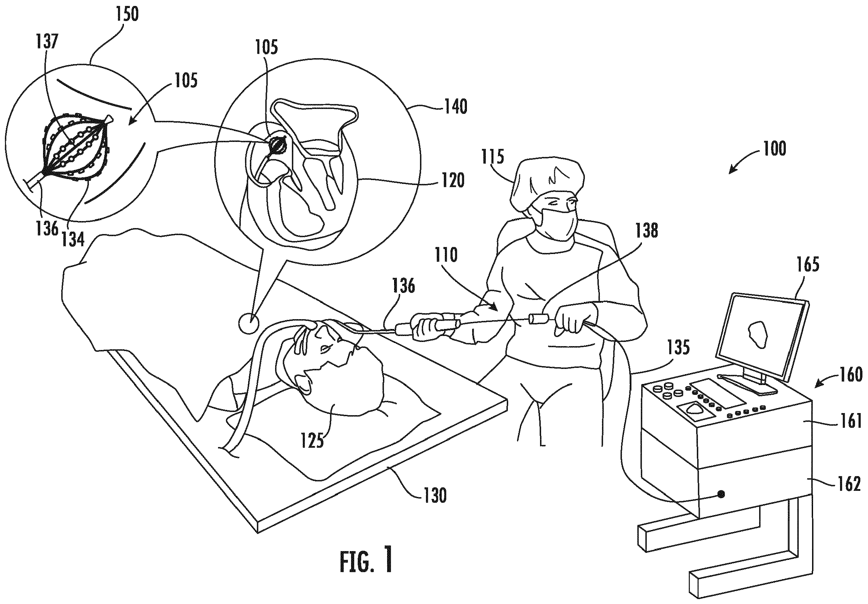

FIG. 1 is a diagram of an exemplary system 100 (e.g., medical device equipment) in which one or more features of the disclosure subject matter can be implemented. All or parts of system 100 may be used to collect information for an imaging dataset (e.g., a training dataset) and/or all or parts of system 100 may be used to implement the machine learning algorithm and the evaluation engine described herein.

The system 100 may include components, such as a catheter 105, that are configured to use intravascular ultrasound and/or MRI catheterization to image of an intra-body organ. The catheter 105 may also be further configured to obtain biometric data including the electrical signals of the heart (e.g., data at a multiplicity of points, such as WACA ablation points). Although the catheter 105 is shown to be a point catheter, it will be understood that a catheter of any shape that includes one or more elements (e.g., electrodes, tracking coils, piezoelectric transducer, etc.) may be used to implement the embodiments disclosed herein.

The system 100 includes a probe 110, having shafts that may be navigated by a physician or a medical professional 115 into a body part, such as a heart 120, of a patient 125 lying on a bed (or a table) 130. According to embodiments, multiple probes may be provided; however, for purposes of conciseness, a single probe 110 is described herein. Yet, it is understood that the probe 110 may represent multiple probes.

The exemplary system 100 can be utilized to detect, diagnose, and treat cardiac conditions (e.g., using the evaluation engine). Cardiac conditions, such as cardiac arrhythmias (atrial fibrillation in particular), persist as common and dangerous medical ailments, especially in the aging population. In patients (e.g., the patient 125) with normal sinus rhythm, the heart (e.g., the heart 120), which includes atrial, ventricular, and excitatory conduction tissue, is electrically excited to beat in a synchronous, patterned fashion (note that this electrical excitement can be detected as intracardiac signals or the like).

In patients (e.g., the patient 125) with cardiac arrhythmias, abnormal regions of cardiac tissue do not follow the synchronous beating cycle associated with normally conductive tissue as in patients with normal sinus rhythm. Instead, the abnormal regions of cardiac tissue aberrantly conduct to adjacent tissue, thereby disrupting the cardiac cycle into an asynchronous cardiac rhythm (note that this asynchronous cardiac rhythm can also be detected as intracardiac signals). Such abnormal conduction has been previously known to occur at various regions of the heart (e.g., the heart 120), for example, in the region of the sino-atrial (SA) node, along the conduction pathways of the atrioventricular (AV) node, or in the cardiac muscle tissue forming the walls of the ventricular and atrial cardiac chambers.

Further, cardiac arrhythmias, including atrial arrhythmias, may be of a multiwavelet reentrant type, characterized by multiple asynchronous loops of electrical impulses that are scattered about the atrial chamber and are often self-propagating (e.g., another example of intracardiac signals). Alternatively, or in addition to the multiwavelet reentrant type, cardiac arrhythmias may also have a focal origin, such as when an isolated region of tissue in an atrium fires autonomously in a rapid, repetitive fashion (e.g., another example of the intracardiac signals). Ventricular tachycardia (V-tach or VT) is a tachycardia, or fast heart rhythm that originates in one of the ventricles of the heart. This is a potentially life-threatening arrhythmia because it may lead to ventricular fibrillation and sudden death.

One type of arrhythmia, atrial fibrillation, occurs when the normal electrical impulses (e.g., another example of the intracardiac signals) generated by the sinoatrial node are overwhelmed by disorganized electrical impulses (e.g., the signal interference) that originate in the atria and pulmonary veins causing irregular impulses to be conducted to the ventricles. An irregular heartbeat results and may last from minutes to weeks, or even years. Atrial fibrillation (AF) is often a chronic condition that leads to a small increase in the risk of death often due to strokes. The first line of treatment for AF is medication that either slows the heart rate or revert the heart rhythm back to normal. Additionally, persons with AF are often given anticoagulants to protect them from the risk of stroke. The use of such anticoagulants comes with its own risk of internal bleeding. In some patients, medication is not sufficient and their AF is deemed to be drug-refractory, i.e., untreatable with standard pharmacological interventions. Synchronized electrical cardioversion may also be used to convert AF to a normal heart rhythm. Alternatively, AF patients are treated by catheter ablation.

A catheter ablation-based treatment may include mapping the electrical properties of heart tissue, especially the endocardium and the heart volume, and selectively ablating cardiac tissue by application of energy. Cardiac mapping (which is an example of heart imaging) includes creating a map of electrical potentials (e.g., a voltage map) of the wave propagation along the heart tissue or a map of arrival times (e.g., a local time activation (LAT) map) to various tissue located points. Cardiac mapping (e.g., a cardiac map) may be used for detecting local heart tissue dysfunction. Ablations, such as those based on cardiac mapping, can cease or modify the propagation of unwanted electrical signals from one portion of the heart to another.

The ablation process damages the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure-mapping followed by ablation-electrical activity at points within the heart is typically sensed and measured by advancing a catheter (e.g., the catheter 105) containing one or more electrical sensors (e.g., the at least one ablation electrode 134 of the catheter 105) into the heart (e.g., the heart 120), and acquiring data at a multiplicity of points. This data (e.g., the WACA ablation points) is then utilized to select the endocardial target areas, at which ablation is to be performed. Note that, due to the use of the evaluation engine employed by the exemplary system 100 (e.g., medical device equipment), the data at the multiplicity of points (i.e., the WACA ablation points) is manipulated into improved image data of cardiac tissue that includes locations within a right or a left WACA, classifications into nine anatomical structures, determinations as good or correct, and predictions of acute reconnections and redo-locations. The improved image data can also include stability measurements (e.g., extracted from x, y, z positions during the ablation) and ablation characteristics (e.g., power, impedance, impedance drop, stability, etc.).

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems in order to reconstruct the anatomy of the heart chamber of interest. In this regard, the evaluation engine employed by the exemplary system 100 (e.g., medical device equipment) herein manipulates and evaluates the data at the multiplicity of points (e.g., the WACA ablation points) to produce improved image data of cardiac tissue so that improved images, scans, and/or maps, along with predictions, for treating cardiac conditions can be generated.

For example, cardiologists rely upon software, such as the Complex Fractionated Atrial Electrograms (CFAE) module of the CARTO^{®} 3 3D mapping system, produced by Biosense Webster, Inc. (Diamond Bar, Calif.), to generate and analyze intracardiac electrograms (EGM). The evaluation engine of the exemplary system 100 (e.g., medical device equipment) enhances this software to generate and analyze improved intracardiac images, scans, and/or maps so that the ablation points can be determined for treatment of a broad range of cardiac conditions, including atrial fibrillation. The improved images, scans, and/or maps supported by the evaluation engine can provide multiple pieces of information regarding the electrophysiological properties of the intra-body organ (e.g., heart and/or organic tissue including the scar tissue) that represent the cardiac substrates (anatomical and functional) of these challenging arrhythmias.

Cardiomyopathies with different etiologies (ischemic, dilated cardiomyopathy (DCM), hypertrophic cardiomyopathy (HCM), arrhythmogenic right ventricular dysplasia (ARVD), left ventricular non-compaction (LVNC), etc.) have an identifiable substrate, featured by areas of unhealthy tissue surrounded by areas of normally functioning cardiomyocytes.

Abnormal tissue is generally characterized by low-voltage EGMs. However, initial clinical experience in endo-epicardial mapping indicates that areas of low-voltage are not always present as the sole arrhythmogenic mechanism in such patients. In fact, areas of low or medium voltage may exhibit EGM fragmentation and prolonged activities during sinus rhythm, which corresponds to the critical isthmus identified during sustained and organized ventricular arrhythmias, e.g., applies only to non-tolerated ventricular tachycardias. Moreover, in many cases, EGM fragmentation and prolonged activities are observed in the regions showing a normal or near-normal voltage amplitude (>1-1.5 mV). Although the latter areas may be evaluated according to the voltage amplitude, they cannot be considered as normal according to the intracardiac signal, thus representing a true arrhythmogenic substrate. The 3D mapping may be able to localize the arrhythmogenic substrate on the endocardial and/or epicardial layer of the right/left ventricle, which may vary in distribution according to the extension of the main disease.

The substrate linked to these cardiac conditions is related to the presence of fragmented and prolonged EGMs in the endocardial and/or epicardial layers of the ventricular chambers (right and left). The 3D mapping system, such as CARTO^{®} 3, is able to localize the potential arrhythmogenic substrate of the cardiomyopathy in terms of abnormal EGM detection.

Electrode catheters (e.g., the catheter 105) are use in medical practice. Electrode catheters are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having at least one electrode at its distal end, into a heart chamber. A reference electrode is provided, generally taped to the skin of the patient or by means of a second catheter that is positioned in or near the heart. Radio frequency (RF) current is applied to the tip electrode of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the electrode also occurs as a result of conduction from the heated tissue to the electrode itself. If the electrode temperature becomes sufficiently high, possibly above 60 degrees Celsius, a thin transparent coating of dehydrated blood protein can form on the surface of the electrode. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs, and the catheter must be removed from the body and the tip electrode cleaned.

Treatments for cardiac conditions such as cardiac arrhythmia often require obtaining a detailed mapping of cardiac tissue, chambers, veins, arteries and/or electrical pathways. For example, a prerequisite for performing a catheter ablation successfully is that the cause of the cardiac arrhythmia is accurately located in the heart chamber. Such locating may be done via an electrophysiological investigation during which electrical potentials are detected spatially resolved with a mapping catheter introduced into the heart chamber. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time. In this case, the evaluation engine can be directly stored and executed by the catheter 105.

Mapping of cardiac areas such as cardiac regions, tissue, veins, arteries and/or electrical pathways of the heart (e.g., 120) may result in identifying problem areas such as scar tissue, arrhythmia sources (e.g., electric rotors), healthy areas, and the like. Cardiac areas may be mapped such that a visual rendering of the mapped cardiac areas is provided using a display, as further disclosed herein. Additionally, cardiac mapping (which is an example of heart imaging) may include mapping based on one or more modalities such as, but not limited to local activation time (LAT), an electrical activity, a topology, a bipolar mapping, a dominant frequency, or an impedance. Data corresponding to multiple modalities may be captured using a catheter inserted into a patient's body and may be provided for rendering at the same time or at different times based on corresponding settings and/or preferences of a medical professional.

Cardiac mapping may be implemented using one or more techniques. As an example of a first technique, cardiac mapping may be implemented by sensing an electrical property of heart tissue, for example, LAT, as a function of the precise location within the heart. The corresponding data may be acquired with one or more catheters that are advanced into the heart using catheters that have electrical and location sensors in their distal tips. As specific examples, location and electrical activity may be initially measured on about 10 to about 20 points on the interior surface of the heart. These data points may be generally sufficient to generate a preliminary reconstruction or map of the cardiac surface to a satisfactory quality. The preliminary map may be combined with data taken at additional points in order to generate a more comprehensive map of the heart's electrical activity. In clinical settings, it is not uncommon to accumulate data at 100 or more sites to generate a detailed, comprehensive map of heart chamber electrical activity. The generated detailed map may then serve as the basis for deciding on a therapeutic course of action, for example, tissue ablation, to alter the propagation of the heart's electrical activity and to restore normal heart rhythm.

Returning to FIG. 1, to implement the noted heart imaging, the medical professional 115 may insert a shaft 137 through a sheath 136, while manipulating a distal end of the shaft 137 using a manipulator 138 near the proximal end of the catheter 105 and/or deflection from the sheath 136. As shown in an inset 140, the catheter 105 may be fitted at the distal end of the shaft 137. The catheter 105 may be inserted through the sheath 136 in a collapsed state and may be then expanded within the heart 120. The catheter 105 may include at least one ablation electrode 134 and a catheter needle, as further disclosed herein.

According to embodiments, the catheter 105 may be configured to ablate tissue areas of a cardiac chamber of the heart 120. Inset 150 shows the catheter 105 in an enlarged view, inside a cardiac chamber of the heart 120. As shown, the catheter 105 may include the at least one ablation electrode 134 coupled onto the body of the catheter. According to other embodiments, multiple elements may be connected via splines that form the shape of the catheter 105. One or more other elements (not shown) may be provided and may be any elements configured to ablate or to obtain biometric data and may be electrodes, transducers, or one or more other elements.

According to embodiments disclosed herein, the ablation electrodes, such as the at least one ablation electrode 134, may be configured to provide energy to tissue areas of an intra-body organ such as heart 120. The energy may be thermal energy and may cause damage to the tissue area starting from the surface of the tissue area and extending into the thickness of the tissue area.

According to embodiments disclosed herein, biometric data may include one or more of LATs, electrical activity, topology, bipolar mapping, dominant frequency, impedance, or the like. The LAT may be a point in time of a threshold activity corresponding to a local activation, calculated based on a normalized initial starting point. Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequency that is prevalent at a portion of a body part and may be different in different portions of the same body part. For example, the dominant frequency of a PV of a heart may be different than the dominant frequency of the right atrium of the same heart. Impedance may be the resistance measurement at a given area of a body part.

As shown in FIG. 1, the probe 110 and the catheter 105 may be connected to a console 160. The console 160 may include a computing device 161, which employs the machine learning algorithm and the evaluation engine as described herein. According to an embodiment, the console 160 and/or the computing device 161 include at least a processor and a memory, where the processor executes computer instructions with respect the machine learning algorithm and the evaluation engine described herein and the memory stores the instructions for execution by the processor.

The computing device 161 can be any computing device including software and/or hardware, such as a general-purpose computer, with suitable front end and interface circuits 162 for transmitting and receiving signals to and from the catheter 105, as well as for controlling the other components of system 100. The computing device 161 may include real-time noise reduction circuitry typically configured as a field programmable gate array (FPGA), followed by an analog-to-digital (A/D) electrocardiogram (ECG) or electrocardiograph or electromyogram (EMG) signal conversion integrated circuit. The computing device 161 may pass the signal from an A/D ECG or EMG circuit to another processor and/or can be programmed to perform one or more functions disclosed herein. For example, the one or more functions include receiving effective points respective to cardiac tissue of a patient, determining an anatomical structural classification for each of the effective points based on a structural segmentation for the cardiac tissue, and providing the anatomical structural classification with the each of the plurality of effective pointsto support treatment of the cardiac tissue. The front end and interface circuits 162 include input/output (I/O) communication interfaces that enables the console 160 to receive signals from and/or transfer signals to the at least one ablation electrode 134.

In some embodiments, the computing device 161 may be further configured to receive biometric data, such as electrical activity, and determine if a given tissue area conducts electricity. According to an embodiment, the computing device 161 may be external to the console 160 and may be located, for example, in the catheter, in an external device, in a mobile device, in a cloud-based device, or may be a standalone processor.

As noted above, the computing device 161 may include a general-purpose computer, which may be programmed in software to carry out the functions of the machine learning algorithm and the evaluation engine described herein. The software may be downloaded to the general-purpose computer in electronic form, over a network, for example, or it may, alternatively or additionally, be provided and/or stored on non-transitory tangible media, such as magnetic, optical, or electronic memory (e.g., any suitable volatile and/or non-volatile memory, such as random-access memory or a hard disk drive). The example configuration shown in FIG. 1 may be modified to implement the embodiments disclosed herein. The disclosed embodiments may similarly be applied using other system components and settings. Additionally, system 100 may include additional components, such as elements for sensing electrical activity, wired or wireless connectors, processing and display devices, or the like.

According to an embodiment, a display 165 is connected to the computing device 161. During a procedure, the computing device 161 may facilitate the presentation of a body part rendering to the medical professional 115 on a display 165, and store data representing the body part rendering in a memory. In some embodiments, the medical professional 115 may be able to manipulate the body part rendering using one or more input devices such as a touch pad, a mouse, a keyboard, a gesture recognition apparatus, or the like. For example, an input device may be used to change a position of the catheter 105, such that rendering is updated. In alternative embodiments, the display 165 may include a touchscreen that can be configured to accept inputs from the medical professional 115, in addition to presenting the body part rendering. Note that the display 165 may be located at a same location or a remote location such as a separate hospital or in separate healthcare provider networks. Additionally, the system 100 may be part of a surgical system that is configured to obtain anatomical and electrical measurements of a patient's organ, such as the heart 120, and performing a cardiac ablation procedure. An example of such a surgical system is the Carto^{®} system sold by Biosense Webster.

The console 160 may be connected, by a cable, to body surface electrodes, which may include adhesive skin patches that are affixed to the patient 125. The processor, in conjunction with a current tracking module, may determine position coordinates of the catheter 105 inside the body part (e.g., the heart 120) of the patient 125. The position coordinates may be based on impedances or electromagnetic fields measured between the body surface electrodes and the electrode or other electromagnetic components (e.g., the at least one ablation electrode 134) of the catheter 105. Additionally, or alternatively, location pads may be located on a surface of bed 130 and may be separate from the bed 130.

The system 100 may also, and optionally, obtain biometric data such as anatomical measurements of the heart 120 using ultrasound, computed tomography (CT), MRI, or other medical imaging techniques known in the art. The system 100 may obtain ECGs or electrical measurements using catheters or other sensors that measure electrical properties of the heart 120. The biometric data including anatomical and electrical measurements may then be stored in a non-transitory tangible media of the console 160. The biometric data may be transmitted to the computing device 161 from the non-transitory tangible media. Alternatively, or in addition, the biometric data may be transmitted to a server, which may be local or remote, using a network as further described herein.

According to one or more embodiments, catheters containing position sensors may be used to determine the trajectory of points on the cardiac surface. These trajectories may be used to infer motion characteristics such as the contractility of the tissue. Maps depicting such motion characteristics may be constructed when the trajectory information is sampled at a sufficient number of points in the heart 120.

Electrical activity at a point in the heart 120 may be typically measured by advancing the catheter 105 containing an electrical sensor at or near its distal tip (e.g., the at least one ablation electrode 134) to that point in the heart 120, contacting the tissue with the sensor and acquiring data at that point. One drawback with mapping a cardiac chamber using the catheter 105 containing only a single, distal tip electrode is the long period of time required to accumulate data on a point-by-point basis over the requisite number of points required for a detailed map of the chamber as a whole. Accordingly, multiple-electrode catheters have been developed to simultaneously measure electrical activity at multiple points in the heart chamber.

Multiple-electrode catheters may be implemented using any applicable shape such as a linear catheter with multiple electrodes, a balloon catheter including electrodes dispersed on multiple spines that shape the balloon, a lasso or loop catheter with multiple electrodes, or any other applicable shape (e.g., a Thermocool SmartTouch irrigated tip CF-sensing ablation catheter). Linear catheters may be fully or partially elastic such that it can twist, bend, and or otherwise change its shape based on received signal and/or based on application of an external force (e.g., cardiac tissue) on the linear catheter. The balloon catheter may be designed such that when deployed into a patient's body, its electrodes may be held in intimate contact against an endocardial surface. As an example, a balloon catheter may be inserted into a lumen, such as a PV. The balloon catheter may be inserted into the PV in a deflated state such that the balloon catheter does not occupy its maximum volume while being inserted into the PV. The balloon catheter may expand while inside the PV such those electrodes on the balloon catheter are in contact with an entire circular section of the PV. Such contact with an entire circular section of the PV, or any other lumen, may enable efficient mapping and/or ablation.

According to an example, a multi-electrode catheter may be advanced into a chamber of the heart 120. Anteroposterior (AP) and lateral fluorograms may be obtained to establish the position and orientation of each of the electrodes. EGMs may be recorded from each of the electrodes in contact with a cardiac surface relative to a temporal reference such as the onset of the P-wave in sinus rhythm from a body surface ECG. The system, as further disclosed herein, may differentiate between those electrodes that register electrical activity and those that do not due to absence of close proximity to the endocardial wall. After initial EGMs are recorded, the catheter may be repositioned, and fluorograms and EGMs may be recorded again. An electrical map may then be constructed from iterations of the process above.

According to an example, cardiac mapping may be generated based on detection of intracardiac electrical potential fields. A non-contact technique to simultaneously acquire a large amount of cardiac electrical information may be implemented. For example, a catheter having a distal end portion may be provided with a series of sensor electrodes distributed over its surface and connected to insulated electrical conductors for connection to signal sensing and processing means. The size and shape of the end portion may be such that the electrodes are spaced substantially away from the wall of the cardiac chamber. Intracardiac potential fields may be detected during a single cardiac beat. According to an example, the sensor electrodes may be distributed on a series of circumferences lying in planes spaced from each other. These planes may be perpendicular to the major axis of the end portion of the catheter. At least two additional electrodes may be provided adjacent at the ends of the major axis of the end portion. As a more specific example, the catheter may include four circumferences with eight electrodes spaced equiangularly on each circumference. Accordingly, in this specific implementation, the catheter may include at least 34 electrodes (32 circumferential and 2 end electrodes).

According to another example, an electrophysiological cardiac mapping system and technique based on a non-contact and non-expanded multi-electrode catheter may be implemented. EGMs may be obtained with catheters having multiple electrodes (e.g., between 42 to 122 electrodes). According to this implementation, knowledge of the relative geometry of the probe and the endocardium may be obtained such as by an independent imaging modality such as transesophageal echocardiography. After the independent imaging, non-contact electrodes may be used to measure cardiac surface potentials and construct maps therefrom. This technique may include the following steps (after the independent imaging step): (a) measuring electrical potentials with a plurality of electrodes disposed on a probe positioned in the heart 120; (b) determining the geometric relationship of the probe surface and the endocardial surface; (c) generating a matrix of coefficients representing the geometric relationship of the probe surface and the endocardial surface; and (d) determining endocardial potentials based on the electrode potentials and the matrix of coefficients.

According to another example, a technique and apparatus for mapping the electrical potential distribution of a heart chamber may be implemented. An intra-cardiac multi-electrode mapping catheter assembly may be inserted into a patient's heart 120. The mapping catheter assembly may include a multi-electrode array with an integral reference electrode, or, preferably, a companion reference catheter. The electrodes may be deployed in the form of a substantially spherical array. The electrode array may be spatially referenced to a point on the endocardial surface by the reference electrode or by the reference catheter which is brought into contact with the endocardial surface. The preferred electrode array catheter may carry a number of individual electrode sites (e.g., at least 24). Additionally, this example technique may be implemented with knowledge of the location of each of the electrode sites on the array, as well as knowledge of the cardiac geometry. These locations are preferably determined by a technique of impedance plethysmography.

According to another example, a heart mapping catheter assembly may include an electrode array defining a number of electrode sites. The mapping catheter assembly may also include a lumen to accept a reference catheter having a distal tip electrode assembly which may be used to probe the heart wall. The mapping catheter may include a braid of insulated wires (e.g., having 24 to 64 wires in the braid), and each of the wires may be used to form electrode sites. The catheter may be readily positionable in a heart 120 to be used to acquire electrical activity information from a first set of non-contact electrode sites and/or a second set of in-contact electrode sites.

According to another example, another catheter for mapping electrophysiological activity within the heart may be implemented. The catheter body may include a distal tip which is adapted for delivery of a stimulating pulse for pacing the heart or an ablative electrode for ablating tissue in contact with the tip. The catheter may further include at least one pair of orthogonal electrodes to generate a difference signal indicative of the local cardiac electrical activity adjacent the orthogonal electrodes.

According to another example, a process for measuring electrophysiologic data in a heart chamber may be implemented. The method may include, in part, positioning a set of active and passive electrodes into the heart 120, supplying current to the active electrodes, thereby generating an electric field in the heart chamber, and measuring the electric field at the passive electrode sites. The passive electrodes are contained in an array positioned on an inflatable balloon of a balloon catheter. In preferred embodiments, the array is said to have from 60 to 64 electrodes.

According to another example, cardiac mapping may be implemented using one or more ultrasound transducers. The ultrasound transducers may be inserted into a patient's heart 120 and may collect a plurality of ultrasound slices (e.g., two dimensional or three-dimensional slices) at various locations and orientations within the heart 120. The location and orientation of a given ultrasound transducer may be known and the collected ultrasound slices may be stored such that they can be displayed at a later time. One or more ultrasound slices corresponding to the position of a probe (e.g., a treatment catheter) at the later time may be displayed and the probe may be overlaid onto the one or more ultrasound slices.

According to other examples, body patches and/or body surface electrodes may be positioned on or proximate to a patient's body. A catheter with one or more electrodes may be positioned within the patient's body (e.g., within the patient's heart 120) and the position of the catheter may be determined by a system based on signals transmitted and received between the one or more electrodes of the catheter and the body patches and/or body surface electrodes. Additionally, the catheter electrodes may sense biometric data (e.g., LAT values) from within the body of the patient (e.g., within the heart 120). The biometric data may be associated with the determined position of the catheter such that a rendering of the patient's body part (e.g., heart 120) may be displayed and may show the biometric data overlaid on a shape of the body.

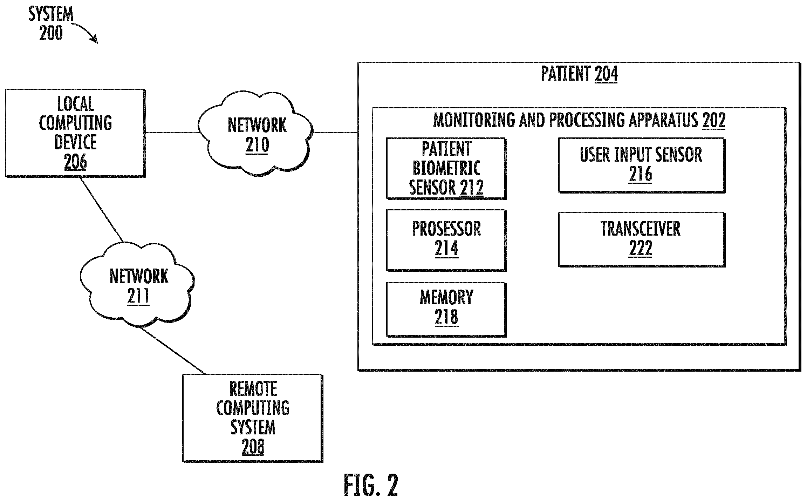

FIG. 2 illustrates a block diagram of an example system 200 for remotely monitoring and communicating biometric data (i.e., patient biometrics, patient data, or patient biometric data) is illustrated. In the example illustrated in FIG. 2, the system 200 includes a monitoring and processing apparatus 202 (i.e., a patient data monitoring and processing apparatus) associated with a patient 204, a local computing device 206, a remote computing system 208, a first network 210, and a second network 211. In accordance with one or more embodiments, the monitoring and processing apparatus 202 can be an example of the catheter 105 of FIG. 1, the patient 204 can be an example of the patient 125 of FIG. 1, and the local computing device 206 can be an example of the console 160 of FIG. 1.

The monitoring and processing apparatus 202 includes a patient biometric sensor 212, a processor 214, a user input (UI) sensor 216, a memory 218, and a transmitter-receiver (i.e., transceiver) 222. In operation, the monitoring and processing apparatus 202 acquires biometric data of the patient 204 (e.g., electrical signals, blood pressure, temperature, blood glucose level or other biometric data) and/or receives at least a portion of the biometric data representing any acquired patient biometrics and additional information associated with any acquired patient biometrics from the one or more other patient biometric monitoring and processing apparatuses. The additional information may be, for example, diagnosis information and/or additional information obtained from an additional device such as a wearable device. The monitoring and processing apparatus 202 may employ the machine learning algorithm and the evaluation engine described herein to process data, including the acquired biometric data as well as any biometric data received from the one or more other patient biometric monitoring and processing apparatuses.

The monitoring and processing apparatus 202 may continually or periodically monitor, store, process, and communicate, via network 210, any number of various patient biometrics (e.g., the acquired biometric data). As described herein, examples of patient biometrics include electrical signals (e.g., ECG signals and brain biometrics), blood pressure data, blood glucose data, and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

The patient biometric sensor 212 may include, for example, one or more transducers configured to convert one or more environmental conditions into an electrical signal, such that different types of biometric data are acquired. For example, the patient biometric sensor 212 may include one or more of an electrode configured to acquire electrical signals (e.g., heart signals, brain signals, or other bioelectrical signals), a temperature sensor (e.g., thermocouple), a blood pressure sensor, a blood glucose sensor, a blood oxygen sensor, a pH sensor, an accelerometer, and a microphone.

As described in more detail herein, the monitoring and processing apparatus 202 may implement the machine learning algorithm and the evaluation engine to receive effective points respective to cardiac tissue of a patient, determine an anatomical structural classification for each of the effective points based on a structural segmentation for the cardiac tissue, and provide the anatomical structural classification with the each of the plurality of effective pointsto support treatment of the cardiac tissue. The monitoring and processing apparatus 202 may implement the machine learning algorithm and the evaluation engine to produce improved image data of cardiac tissue so that improved images, scans, and/or maps, along with predictions, for treating cardiac conditions can be generated.

In another example, the monitoring and processing apparatus 202 may be an ECG monitor for monitoring ECG signals of a heart (e.g., the heart 120 of FIG. 1). In this regard, the patient biometric sensor 212 of the ECG monitor may include one or more electrodes (e.g., electrodes of the catheter 105 of FIG. 1) for acquiring ECG signals. The ECG signals may be used for treatment of various cardiovascular diseases.

In another example, the monitoring and processing apparatus 202 may be a continuous glucose monitor (CGM) for continuously monitoring blood glucose levels of a patient on a continual basis for treatment of various diseases, such as type I and type II diabetes. In this regard, the patient biometric sensor 212 of the CGM may include a subcutaneously disposed electrode (e.g., electrodes of the catheter 105 of FIG. 1), which may monitor blood glucose levels from interstitial fluid of the patient. The CGM may be, for example, a component of a closed-loop system in which the blood glucose data is sent to an insulin pump for calculated delivery of insulin without user intervention.

The processor 214 may be configured to receive, process, and manage, biometric data acquired by the patient biometric sensor 212, and communicate the biometric data to the memory 218 for storage and/or across the network 210 via the transceiver 222. Data from one or more other monitoring and processing apparatus 202 may also be received by the processor 214 through the transceiver 222, as described in more detail herein. Also, as described in more detail herein, the processor 214 may be configured to respond selectively to different tapping patterns (e.g., a single tap or a double tap) received from the UI sensor 216 (e.g., a capacitive sensor therein), such that different tasks of a patch (e.g., acquisition, storing, or transmission of data) may be activated based on the detected pattern. In some embodiments, the processor 214 can generate audible feedback with respect to detecting a gesture.

The UI sensor 216 includes, for example, a piezoelectric sensor or a capacitive sensor configured to receive a user input, such as a tapping or touching. For example, UI sensor 216 may be controlled to implement a capacitive coupling, in response to tapping or touching a surface of the monitoring and processing apparatus 202 by the patient 204. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface, such that the tapping or touching of the surface activates the monitoring device.

The memory 218 is any non-transitory tangible media, such as magnetic, optical, or electronic memory (e.g., any suitable volatile and/or non-volatile memory, such as random-access memory or a hard disk drive).

The transceiver 222 may include a separate transmitter and a separate receiver. Alternatively, the transceiver 222 may include a transmitter and receiver integrated into a single device.

According to an embodiment, the monitoring and processing apparatus 202 may be an apparatus that is internal to a body of the patient 204 (e.g., subcutaneously implantable). The monitoring and processing apparatus 202 may be inserted into the patient 204 via any applicable manner including orally injecting, surgical insertion via a vein or artery, an endoscopic procedure, or a laparoscopic procedure.

According to an embodiment, the monitoring and processing apparatus 202 may be an apparatus that is external to the patient 204. For example, as described in more detail herein, the monitoring and processing apparatus 202 may include an attachable patch (e.g., that attaches to a patient's skin). The monitoring and processing apparatus 202 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, a monitoring and processing apparatus 202 may include both components that are internal to the patient and components that are external to the patient.

While a single monitoring and processing apparatus 202 is shown in FIG. 2, example systems may include a plurality of patient biometric monitoring and processing apparatuses. For instance, the monitoring and processing apparatus 202 may be in communication with one or more other patient biometric monitoring and processing apparatuses. Additionally, or alternatively, the one or more other patient biometric monitoring and processing apparatus may be in communication with the network 210 and other components of the system 200.

The local computing device 206 and/or the remote computing system 208, along with the monitoring and processing apparatus 202, can be any combination of software and/or hardware that individually or collectively store, execute, and implement the machine learning algorithm and the evaluation engine and functions thereof. Further, the local computing device 206 and/or the remote computing system 208, along with the monitoring and processing apparatus 202, can be an electronic, computer framework including and/or employing any number and combination of computing device and networks utilizing various communication technologies, as described herein. The local computing device 206 and/or the remote computing system 208, along with the monitoring and processing apparatus 202, can be easily scalable, extensible, and modular, with the ability to change to different services or reconfigure some features independently of others.

According to an embodiment, the local computing device 206 and the remote computing system 208, along with the monitoring and processing apparatus 202, include at least a processor and a memory, where the processor executes computer instructions with respect the machine learning algorithm and the evaluation engine and the memory stores the instructions for execution by the processor.

The local computing device 206 of system 200 is in communication with the monitoring and processing apparatus 202 and may be configured to act as a gateway to the remote computing system 208 through the second network 211. The local computing device 206 may be, for example, a, smart phone, smartwatch, tablet or other portable smart device configured to communicate with other devices via network 211. Alternatively, the local computing device 206 may be a stationary or standalone device, such as a stationary base station including, for example, modem and/or router capability, a desktop or laptop computer using an executable program to communicate information between the processing apparatus 202 and the remote computing system 208 via the PC's radio module, or a USB dongle. Biometric data may be communicated between the local computing device 206 and the monitoring and processing apparatus 202 using a short-range wireless technology standard (e.g., Bluetooth, Wi-Fi, ZigBee, Z-wave and other short-range wireless standards) via the short-range wireless network 210, such as a local area network (LAN) (e.g., a personal area network (PAN)). In some embodiments, the local computing device 206 may also be configured to display the acquired patient electrical signals and information associated with the acquired patient electrical signals, as described in more detail herein.

In some embodiments, the remote computing system 208 may be configured to receive at least one of the monitored patient biometrics and information associated with the monitored patient via network 211, which is a long-range network. For example, if the local computing device 206 is a mobile phone, network 211 may be a wireless cellular network, and information may be communicated between the local computing device 206 and the remote computing system 208 via a wireless technology standard, such as any of the wireless technologies mentioned above. As described in more detail herein, the remote computing system 208 may be configured to provide (e.g., visually display and/or aurally provide) the at least one of the patient biometrics and the associated information to a medical professional, a physician, a healthcare professional, or the like.

In FIG. 2, the network 210 is an example of a short-range network (e.g., local area network (LAN), or personal area network (PAN)). Information may be sent, via short-range network 210, between the monitoring and processing apparatus 202 and the local computing device 206 using any one of various short-range wireless communication protocols, such as Bluetooth, Wi-Fi, Zigbee, Z-Wave, near field communications (NFC), ultraband, Zigbee, or infrared (IR).

The network 211 may be a wired network, a wireless network or include one or more wired and wireless networks, such as an intranet, a local area network (LAN), a wide area network (WAN), a metropolitan area network (MAN), a direct connection or series of connections, a cellular telephone network, or any other network or medium capable of facilitating communication between the local computing device 206 and the remote computing system 208. Information may be sent, via the network 211 using any one of various long-range wireless communication protocols (e.g., TCP/IP, HTTP, 3G, 4G/LTE, or 5G/New Radio). Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology. Additionally, several networks may work alone or in communication with each other to facilitate communication in the network 211. In some instances, the remote computing system 208 may be implemented as a physical server on the network 211. In other instances, the remote computing system 208 may be implemented as a virtual server a public cloud computing provider (e.g., Amazon Web Services (AWS) ^{®}) of the network 211.

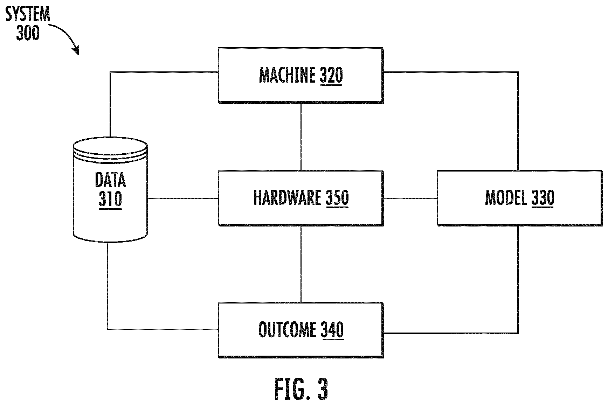

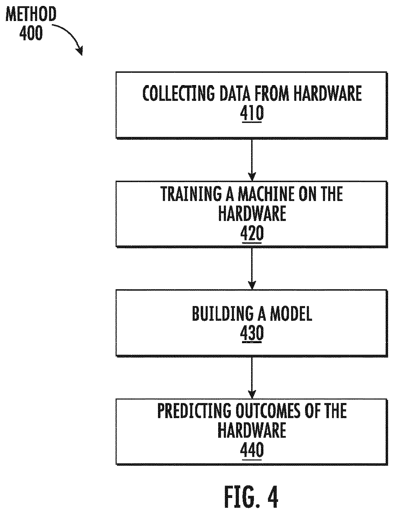

FIG. 3 illustrates a graphical depiction of an artificial intelligence system 300 according to one or more embodiments. The artificial intelligence system 300 includes data 310, a machine 320, a model 330, a plurality of outcomes 340, and underlying hardware 350. FIG. 4 illustrates a block diagram of a method 400 performed in the artificial intelligence system of FIG. 3. The description of FIGS. 3-4 is made with reference to FIG. 2 for ease of understanding.

In general, the artificial intelligence system 300 operates the method 400 by using the data 310 to train the machine 320 (e.g., the local computing device 206 of FIG. 2) while building the model 330 to enable the plurality of outcomes 340 (to be predicted). In such a configuration, the artificial intelligence system 300 may operate with respect to the hardware 350 (e.g., the monitoring and processing apparatus 202 of FIG. 2) to train the machine 320, build the model 330, and predict outcomes using algorithms. These algorithms may be used to solve the trained model 330 and predict outcomes 340 associated with the hardware 350. These algorithms may be divided generally into classification, regression, and clustering algorithms.

At block 410, the method 400 includes collecting the data 310 from the hardware 350. The machine 320 operates as the controller or data collection associated with the hardware 350 and/or is associated therewith. The data 310 (e.g., biometric data, which may originate with the monitoring and processing apparatus 202 of FIG. 2) may be related to the hardware 350. For instance, the data 310 may be on-going data, or output data associated with the hardware 350. The data 310 may also include currently collected data, historical data, or other data from the hardware 350. For example, the data 310 may include measurements during a surgical procedure and may be associated with an outcome of the surgical procedure. For example, a temperature of a heart (e.g., of the patient 204) may be collected and correlated with an outcome of a heart procedure.

At block 420, the method 400 includes training the machine 320, such as with respect to the hardware 350. The training may include an analysis and correlation of the data 310 collected at block 410. For example, in the case of the heart, the data 310 of temperature and outcome may be trained to determine if a correlation or link exists between the temperature of the heart (e.g., of the patient 204) during the heart procedure and the outcome.

At block 430, the method 400 includes building the model 330 on the data 310 associated with the hardware 350. Building the model 330 may include physical hardware or software modeling, algorithmic modeling, and/or the like. This modeling may seek to represent the data 310 that has been collected and trained. According to an embodiment, the model 330 may be configured to model the operation of hardware 350 and model the data 310 collected from the hardware 350 to predict the outcome achieved by the hardware 350. In accordance with one or more embodiments, the model 330, with respect to the evaluation engine, receives effective points respective to cardiac tissue of a patient, determines an anatomical structural classification for each of the effective points based on a structural segmentation for the cardiac tissue, and provides the anatomical structural classification with the each of the plurality of effective pointsto support treatment of the cardiac tissue.

At block 440, the method 400 includes predicting the plurality of outcomes 340 of the model 330 associated with the hardware 350. This prediction of the plurality of outcomes 340 may be based on the trained model 330. For example and to increase understanding of the disclosure, in the case of the heart, if the temperature during the procedure is between 36.5 degrees Celsius and 37.89 degrees Celsius (i.e., 97.7 degrees Fahrenheit and 100.2 degrees Fahrenheit) produces a positive result from the heart procedure, the outcome can be predicted in a given procedure based on the temperature of the heart during the heart procedure. Thus, using the outcome 340 that is predicted, the hardware 350 may be configured to provide a certain desired outcome 340 from the hardware 350.

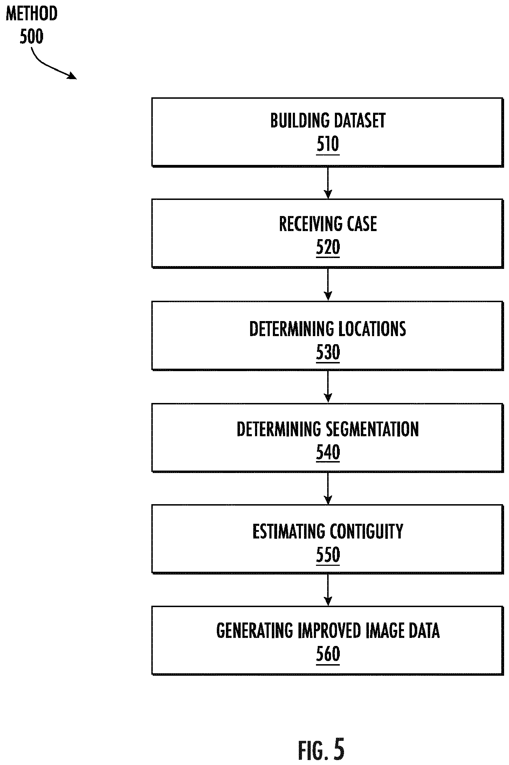

FIG. 5 illustrates a block diagram of a method 500 is illustrated according to one or more embodiments. In accordance with an embodiment, the method 500 is implemented by the evaluation engine. Any combination of software and/or hardware (e.g., the local computing device 206 and the remote computing system 208, along with the monitoring and processing apparatus 202) can individually or collectively store, execute, and implement the evaluation engine and functions thereof.

In general, the evaluation engine provides automatic anatomical segmentation, contiguity estimation, and gap prediction for the effective points to provide improved image data of cardiac tissue. The evaluation engine provides improved image data of cardiac tissue to support, guide, and recommend to the physician on an overall effectiveness of ablation procedure. Anatomical segmentation is an ability by the evaluation engine to determine in which segment of the left and right pulmonary veins that each effective point resides (e.g., left and right WACA detection, and the classification/ segmentation of WACA points into nine anatomical structures). In accordance with one or more embodiments, other structure combinations with respect to or in lieu of the nine anatomical structures are contemplated, such as at least one of left WACA, right WACA, ostial PVI, roof line, left carina, right carina, posterior line, inferior line, mitral isthmus line, anterior line, anterior line, and cavo-tricuspid isthmus, superior vena cava isolation. Contiguity estimation is an ability by the evaluation engine to determine indirect contact between the two separate circular rings around the left and right PVs and between the two separate circular rings and structural elements outside the left and right pulmonary veins. For example, a physician desires to deliver contiguous lesions where a center-to-center distance between two ablation points is ≤6 mm. The evaluation engine investigates an effectiveness of each lesion and estimates a size and depth of each lesion to decide if the lesion is sufficient. Accordingly, the contiguity can be evaluated by the evaluation engine in a local sense, i.e., is the lesion between two ablation points is effective. The evaluation engine also operates in a global sense, i.e., testing all the ablations points in the left (or right) WACA and estimating a possibility for recognizing potential reconnection. Gap prediction is an ability by the evaluation engine to determine spaces between the effective points where PV reconnection is possible. In accordance with one or more embodiments, the evaluation engine can classify/segment all ablation points automatically, such that left and right isthmus or carina ablation lines are detected automatically.

The method 500 begins at block 510, where the evaluation engine builds a dataset that includes annotated anatomical structure of a cardiac tissue (e.g., a human heart), all ablation points with their characteristics (e.g., force, force over time, impedance, impedance drop, duration, x, y, z location), and intracardiac ECG (optional input to all algorithms). The annotated anatomical structure can include three-dimensional data files that may depict the human heart and verified effective points (e.g., data for classifying anatomical structure includes x, y, z, locations of ablation points; actual anatomy, such as mesh files and three-dimensional visualization of an atria; and ablation properties, which can leverage the intracardiac ECG characteristics around ablation points if they exist). In accordance with one or more embodiments, each ablation type can be described by an annotation: potential redo location, potential acute reconnection location, and whether it is an effective ablation point (e.g., yes or no decision for each point).

In accordance with one or more embodiments, the annotated anatomical structures are initially training data built by manual contiguity estimations and annotations that verify the effective points generated by the heart mapping phase. The manual contiguity estimations can be ablation points that were added after the heart mapping phase. The manual annotations can be markings made by medical professionals. In accordance with one or more embodiments, the medical professional can mark locations and/or ablation type. Note that ablation types can include acute reconnection points (e.g., after pacing, a physician identifies the location of reconnection and adds ablation point to isolate the vein), long term reconnection (redo) ablation point (e.g., long after a case ended, the patient has atrial fibrillation and the physician decides to perform another ablation procedure), and an efficient ablation.

At block 520, the evaluation engine receives a case. The case can be received in real-time (or retrospectively), during a heart mapping phase of a WACA session. The case includes effective points mapping cardiac tissue, where the effective points make two separate circular rings around the left and right PVs. The case is evaluated by the evaluation engine. Note that, for example, the evaluation engine can be provided an aided tool to retrospective analysis of physician decisions to improve his or his colleagues work.

At block 530, the evaluation engine determines right and left WACA locations for each effective point of the received case. For instance, a sub-algorithm of the evaluation engine is used to automatically compare location information of the effective points to dimensional information along an axis (e.g., using X-axis with a first range for a left location and a second range for a right location). In turn, the effective points are divided based on the comparison.

At block 540, the evaluation engine determines an anatomical structural classification for each effective point of the received case (e.g., based on manual annotations of the training data, using the dataset from block 510; segment/group similar ablation points with the same anatomical code and associate them to the same anatomical structure). Note that a WACA anatomical structure code as further described herein.

At block 550, the evaluation engine estimates contiguity of the two separate circular rings around the left and right PVs. The output of this estimation can include a probability for contiguity per effective point. In accordance with one or more embodiments, each effective point is denoted by 0 or 1, where 0 represent that a "bad" ablation point and 1 represents a "good" ablation point. Additionally, distances of each effective point to a next ablation and a previous ablation in the ring can contribute to the "bad/good" determination.

At block 560, the evaluation engine generates the improved image data for the cardiac tissue being mapped (e.g., by merging the estimations of block 550 and the determinations of blocks 530 and 540). In turn, the improved image data can be used to predict acute reconnections and redo-locations, so that appropriate action can be taken (by the physician or the medical professional) during the ablation phase in real time.

In accordance with one or more embodiments, the training dataset is utilized by the evaluation engine to automatically generate contiguity estimations and annotations on the annotated anatomical structure, such that machine learned data is created and added to the dataset. The machine learned data can become a majority of the dataset with respect to the training data, and tagged data based on feedback from medical experts (e.g., tagged ablation types and/or anatomical structure types) can be added to the dataset.

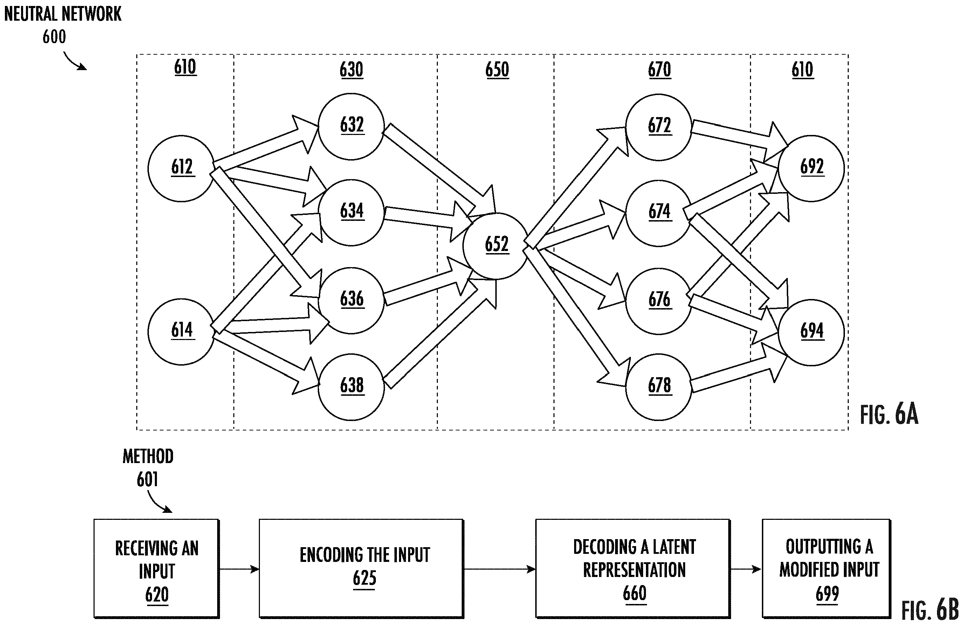

FIG. 6A illustrates an example of an autoencoder architecture 600 and FIG. 6B illustrates a block diagram of a method 601 performed in the autoencoder architecture 600. The autoencoder architecture 600 operates to support implementation of the machine learning algorithm and the evaluation engine described herein. The autoencoder architecture 600 can be implemented in hardware, such as the machine 320 (e.g., the local computing device 206 of FIG. 2) and/or the hardware 350 (e.g., the monitoring and processing apparatus 202 of FIG. 2). Modules 610, 630, 650 of autoencoder 600 collectively operate as a neural network that perform the encoding portion of the autoencoder 600. Modules 750, 670, 610 of autoencoder 600 collectively operate as a neural network that performs the decoding portion of the autoencoder 600. In general, a neural network is a network or circuit of neurons, or in a modern sense, an artificial neural network (ANN), composed of artificial neurons or nodes or cells.

For example, an ANN involves a network of processing elements (artificial neurons) which can exhibit complex global behavior, determined by the connections between the processing elements and element parameters. These connections of the network or circuit of neurons are modeled as weights. A positive weight reflects an excitatory connection, while negative values mean inhibitory connections. Inputs are modified by a weight and summed using a linear combination. An activation function may control the amplitude of the output. For example, an acceptable range of output is usually between 0 and 1, or it could be -1 and 1.

In most cases, the ANN is an adaptive system that changes its structure based on external or internal information that flows through the network. In more practical terms, neural networks are non-linear statistical data modeling or decision-making tools that can be used to model complex relationships between inputs and outputs or to find patterns in data. Thus, ANNs may be used for predictive modeling and adaptive control applications, while being trained via a dataset. Note that self-learning resulting from experience can occur within ANNs, which can derive conclusions from a complex and seemingly unrelated set of information. The utility of artificial neural network models lies in the fact that they can be used to infer a function from observations and also to use it. Unsupervised neural networks can also be used to learn representations of the input that capture the salient characteristics of the input distribution, and more recently, deep learning algorithms, which can implicitly learn the distribution function of the observed data. Learning in neural networks is particularly useful in applications where the complexity of the data or task makes the design of such functions by hand impractical.

Neural networks can be used in different fields. The tasks to which ANNs are applied tend to fall within the following broad categories: function approximation, or regression analysis, including time series prediction and modeling; classification, including pattern and sequence recognition, novelty detection and sequential decision making, data processing, including filtering, clustering, blind signal separation and compression.

Application areas of ANNs include nonlinear system identification and control (vehicle control, process control), game-playing and decision making (backgammon, chess, racing), pattern recognition (radar systems, face identification, object recognition), sequence recognition (gesture, speech, handwritten text recognition), medical diagnosis, financial applications, data mining (or knowledge discovery in databases, "KDD"), visualization and e-mail spam filtering. For example, it is possible to create a semantic profile of user's interests emerging from pictures trained for object recognition.

According to one or more embodiments, the neural network 600 implements a long short-term memory neural network architecture, a CNN architecture, or other the like. The neural network 600 can be configurable with respect to a number of layers, a number of connections (e.g., encoder/decoder connections), a regularization technique (e.g., dropout); and an optimization feature.

The long short-term memory neural network architecture includes feedback connections and can process single data points (e.g., such as images), along with entire sequences of data (e.g., such as speech or video). A unit of the long short-term memory neural network architecture can be composed of a cell, an input gate, an output gate, and a forget gate, where the cell remembers values over arbitrary time intervals and the gates regulate a flow of information into and out of the cell.

The CNN architecture is a shared-weight architecture with translation invariance characteristics where each neuron in one layer is connected to all neurons in the next layer. The regularization technique of the CNN architecture can take advantage of the hierarchical pattern in data and assemble more complex patterns using smaller and simpler patterns. If the neural network 600 implements the CNN architecture, other configurable aspects of the architecture can include a number of filters at each stage, kernel size, a number of kernels per layer.

As shown in FIG. 6B, the method 601 depicts operations of the neural network 600 (e.g., an autoencoder of the evaluation engine). In the neural network 600, an input layer 610 is represented by a plurality of inputs, such as 612 and 614. With respect to block 620 of the method 601, the input layer 610 receives the plurality of inputs (e.g., data at a multiplicity of points, such as WACA points) as an initial operation. The plurality of inputs can be ultrasound signals, radio signals, audio signal, or a two- or three-dimensional image/model. More particularly, the plurality of inputs can be represented as input data (X), which is raw data recorded from the heart mapping phase of a WACA session.

At block 625 of the method 601, the neural network 600 encodes the plurality of inputs utilizing an intracardiac dataset (e.g., the dataset produced by the evaluation engine in block 510 of FIG. 5) to produce a latent representation. The latent representation includes one or more intermediary images derived from the plurality of inputs. According to one or more embodiments, the latent representation is generated by an element-wise activation function (e.g., a sigmoid function or a rectified linear unit) of the autoencoder of the evaluation engine that applies a weight matrix to the input intracardiac signals and adds a bias vector to the result. Note that weights and biases of the weight matrix and the bias vector can be initialized randomly, and then updated iteratively during training.

As shown in FIG. 6A, the inputs 612 and 614 are provided to a hidden layer 630 depicted as including nodes 632, 634, 636, and 638. Thus, layers 610, 630, and 650 can be considered an encoder stage that takes the plurality of inputs 612 and 614 and transfer it to a deep neural network depicted in 630 to learn some smaller representation of the input (e.g., a resulting the latent representation or data coding 652). The deep neural network could be CNNs, a long short-term memory neural network, a fully connected neural network, or combination thereof. The inputs 612 and 614 can be intracardiac ECG, ECG, or intracardiac ECG and ECG. This encoding provides a dimensionality reduction of the input intracardiac signals. Dimensionality reduction is a process of reducing the number of random variables (of the plurality of inputs) under consideration by obtaining a set of principal variables. For instance, dimensionality reduction can be a feature extraction that transforms data (e.g., the plurality of inputs) from a high-dimensional space (e.g., more than 10 dimensions) to a lower-dimensional space (e.g., 2-3 dimensions). The technical effects and benefits of dimensionality reduction include reducing time and storage space requirements for the data, improving visualization of the data, and improving parameter interpretation for machine learning. This data transformation can be linear or nonlinear. The operations of receiving (block 620) and encoding (block 625) can be considered a data preparation portion of the multi-step data manipulation by the autoencoder of the evaluation engine.

At block 660 of the method 610, the neural network 600 decodes the latent representation to produce output intracardiac signals. The decoding stage takes the encoder output (e.g., the resulting the latent representation or data coding 652) and tries to reconstruct using another deep neural network 660 some form of the inputs 612 and 614. In this regard, the nodes 672, 674, 676, and 678 are combined to produce in the output layer 690 outputs 692 and 694, as shown in block 699. That is, the output layer 690 reconstructs the inputs 612 and 614 on a reduced dimension but without the signal interferences, signal artifacts, and signal noise. Examples of the outputs 692 and 694 include Intracardiac ECG, clean version of intracardiac ECG (denoised version), ECG, and denoised ECG. The denoised version of intracardiac ECG could be free from one or more of the following: far field reduction, power line noise, contact noise, deflection noise, baseline wonders, respiration noise, and Fluro noise.

The neural network 600 performs the processing via the hidden layer 630 of the nodes 632, 634, 636, and 638 to exhibit complex global behavior, determined by the connections between the processing elements and element parameters. The target data for the output layer 650 includes target data type one ventricular activity (Y1) and includes target data type two input data after far field reduction (Y2).

In accordance with an embodiment, the autoencoder of the evaluation engine can be a denoising autoencoder to find mapping functions (f, g) such that f(X) = Y1 and g(X) = Y2. Any combination of software and/or hardware (e.g., the local computing device 206 and the remote computing system 208, along with the monitoring and processing apparatus 202) can individually or collectively store, execute, and implement the denoising autoencoder and functions thereof. The denoising autoencoder trains the autoencoder to reconstruct an input from a corrupted version of itself to force the hidden layer (e.g., the hidden layer 630 of FIG. 6) to discover more robust features (i.e., useful features that will constitute better higher level representations of the input) and prevent it from learning a particular identity (i.e., always returning to a same value). In this regard, the denoising autoencoder encodes the input (e.g., to preserve information about the input) and reverses the effect of a corruption process stochastically applied to the input of the autoencoder.

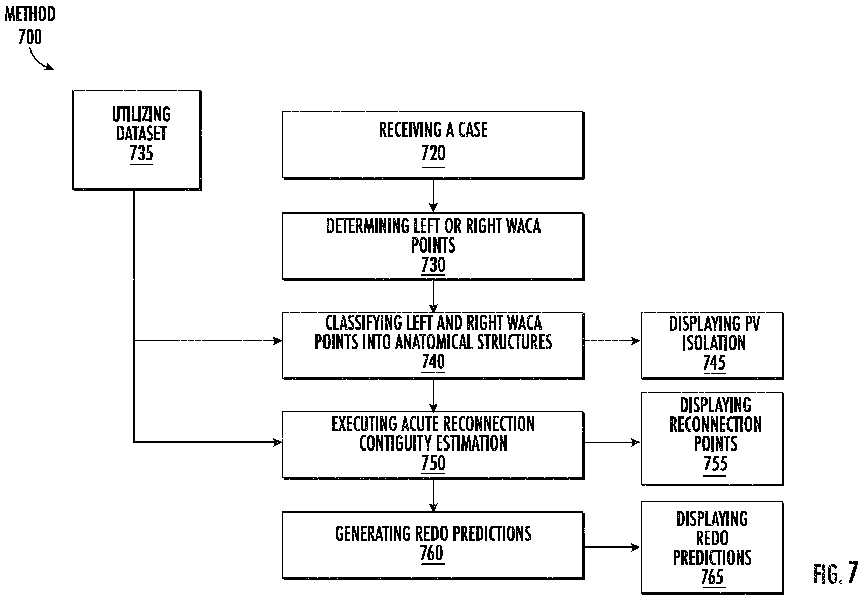

FIG. 7 illustrates a block diagram of a method 700 according to one or more embodiments. Any combination of software and/or hardware (e.g., the local computing device 206 and the remote computing system 208, along with the monitoring and processing apparatus 202) can individually or collectively store, execute, and implement the method 700 within the context of the evaluation engine and functions thereof. In general, the method 700 can be implemented with respect to a WACA treatment, which includes WACA point mapping and WACA phases. That is, implementing the method 700 with respect to the WACA treatment enables a physician and/or a medical professional to more effectively treat various cardiovascular diseases with ablation itself.

For example, a catheter based WACA treatment utilizing the method 700 maps the electrical properties of the left and right PVs and provides direct information to the physician and/or the medical professional. In this regard, the mapping and the direct information includes automatic anatomical segmentation, contiguity estimation, and gap prediction for the WACA points.

The process flow 700 begins at block 720, where the evaluation engine receives a case. The case can be received in real-time, during the WACA point mapping phase of the WACA treatment. The case includes WACA points mapping the left and right PVs, where the WACA points themselves make two separate circular rings around the left and right PVs.

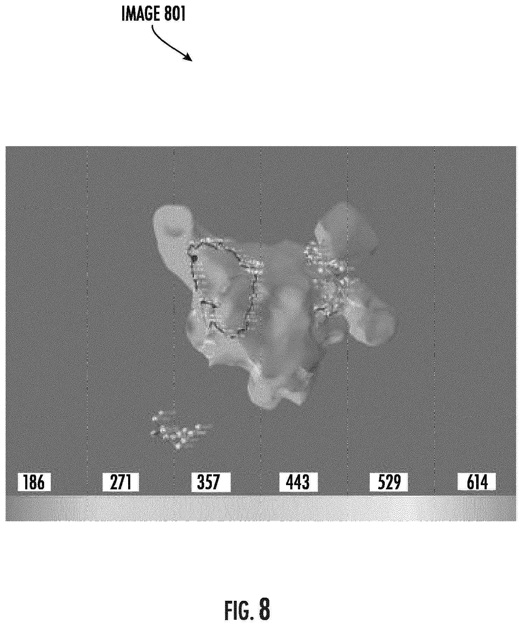

At block 730, the evaluation engine determines right and left WACA locations for each WACA point of the received case or at least one of left WACA, right WACA, ostial PVI, roof line, left carina, right carina, posterior line, inferior line, mitral isthmus line, anterior line, anterior line and cavo-tricuspid isthmus, and superior vena cava isolation lines. For instance, a sub-algorithm of the evaluation engine evaluates the WACA points to estimate left and right WACA rings. The estimation can be considered a feature extraction to determine morphological ring features. In some cases, the evaluation engine can automatically compare location information of the WACA points to dimensional information along an axis (e.g., using X-axis with a first range for a left location and a second range for a right location). Referring also to FIG. 8 illustrating a graphical image 801 is shown according to one or more embodiments. The graphical image 801 is an example of a graphical user interface providing an image of the PVs with two separate circular rings. Each ring includes the WACA points, such as those produced in block 720.

At block 735, the evaluation engine utilizes a dataset. In one example, the dataset includes over 370 cases, each of which comprises manual annotations and/or ablation features (e.g., radio frequency index (RFIndex), force time integral (FTI, how much forced is induced during an ablation point), etc.).

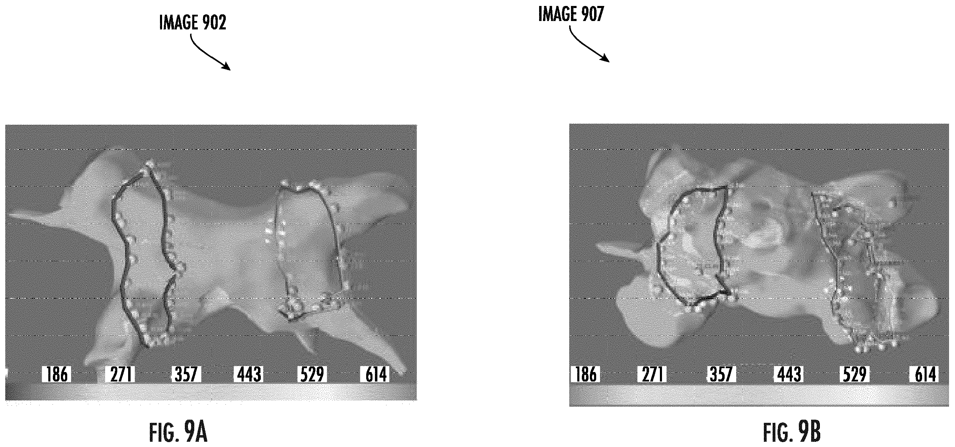

Referring now to FIG. 9A and FIG. 9B illustrates graphical images 902 and 907, respectively, of a user interface showing datasets according to one or more embodiments. The graphical image 902 is an example of manual annotations of nine anatomical structures. The graphical image 907 is an example of manual annotations for ablation type.

At block 740, the evaluation engine determines an anatomical structural classification for each WACA point of the received case (e.g., based on manual annotations of the training data). Anatomical structural classification (e.g., segmentation) is an ability by the evaluation engine to determine in which segment of the left and right pulmonary veins that each WACA point resides. At block 745, the evaluation engine displays the results of the anatomical structural classification. In this regard, the physician or the medical personnel uses the displayed result for more effective treatment of various cardiovascular diseases.

In accordance with one or more embodiments, anatomical structural classification may be defined across nine structures. Table 1 illustrates nine structures or segments: right posterior, right inferior, right roof, right anterior, left posterior, left inferior, left roof, left ridge, and left anterior.

**Table 1**

| **SEGMENT** | **NAME** |
|---|---|
| Right Posterior | RPS |
| Right Inferior | RIN |
| Right Roof | RRF |
| Right Anterior | RAN |
| Left Posterior | LPS |
| Left Inferior | LIN |
| Left Roof | LRF |
| Left Ridge | LRG |
| Left Anterior | LAN |

Each structure or segment may be represented by a unique code, as shown in the name column of Table 1 and/or by a set of numbers (e.g., 1 to 9). Other structure combinations with respect to or in lieu of the nine structures or segments, such as at least one of left WACA, right WACA, ostial PVI, roof line, left carina, right carina, posterior line, inferior line, mitral isthmus line, anterior line, anterior line, and cavo-tricuspid isthmus, superior vena cava isolation, may be utilized.

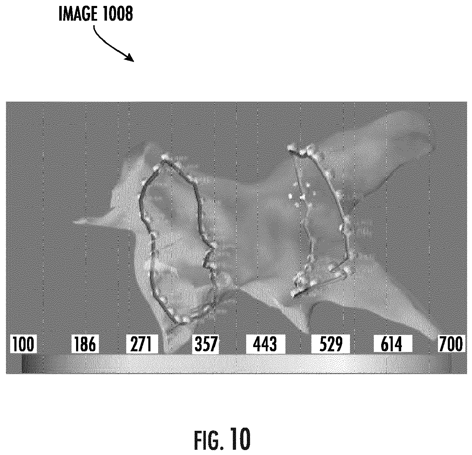

FIG. 10 illustrates a graphical depiction 1008 of classifying left and right WACA points into anatomical structures by manual annotation. The graphical depiction 1008 demonstrates a left atria with ablation points, where the text represents decision of the evaluation engine (if the text is a first color, such as green, the evaluation engine is correct; if the text is a second color, such as red, the evaluation engine is incorrect).

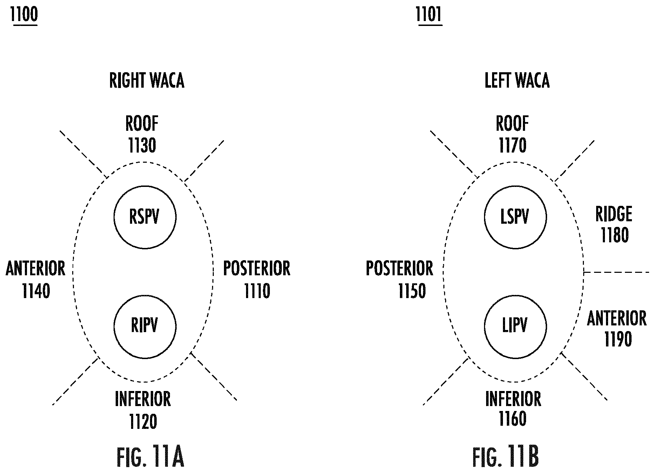

FIG. 11A illustrates a diagram of a right anatomical structure identified as right WACA 1100. Right WACA 1100 includes a posterior region 1110 on the right, an inferior region 1120 below, a roof region 1130 above, and an anterior region 1140 on the left.

FIG. 11B illustrates a diagram of a left anatomical structure identified as left WACA 1101. Left WACA 1101 includes a posterior region 1150 on the left, an inferior region 1160 below, a roof region 1170 above, a ridge region 1180 on the upper right, and an anterior region 1190 on the lower right.

A comparison of the elements of Table 1 to the diagrams 1100, 1101 of FIGs. 11A and 11B shows the nine structures or segments positioned around the right and left PVs.

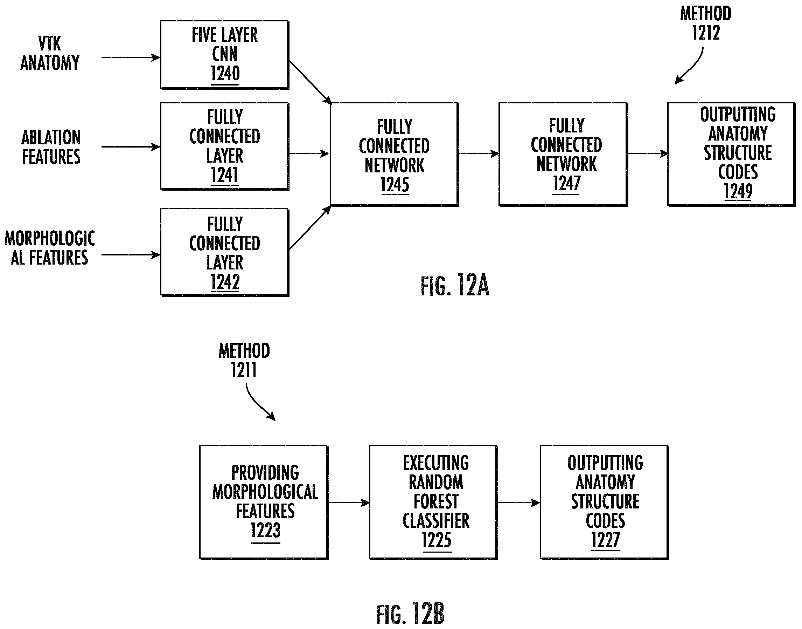

In example operations of block 740, FIG. 12A illustrates a block diagram of a classification method 1212 and FIG. 12B illustrates a block diagram of a classification method 1211. The method 1211 is an example operation of a random forest for acute reconnection classifier (e.g., a random forest classifier). Method 1212 of FIG. 12, as discussed herein, the input to anatomical structure classification may include a set of characteristics/features per ablation point, a 3D representation of the atria/CT scan/ultrasound/fast anatomical mapping/etc. (e.g., VTK file), and IC ECG or body surface ECG. The output 1249 of the anatomical structure classifier is an anatomical structure code k (k = 1... K). In accordance with one or more embodiments, the 3D representation of the atria may be processed using a deep CNN network to obtain an initial estimate of the KxM probability matrix indicating the likelihood that each one of the m's (m = 1, ..., M) ablation points is associated to each one of the k's anatomical structures (k = 1,..,K). The IC ECG may be processed using deep autoencoder to obtain a set of features. All features (and characteristics) may be processed by a set of dense neural networks to anatomical structure predictions.

More particularly, the method 1212 includes leveraging random forest classification, fully connected dense layers, and a CNN architecture. In this way, a five-layer CNN receives (block 1240) an anatomy of a left atria from a VTK file, a fully connected layer receives (block 1241) ablation features, and a fully connected layer receives (block 1242) morphological features, along with ablation features. For example, as described here, ablation positions (x, y, z) locations, and ablation characteristics, are processed with the VTK file using deep CNN network to obtain a set of features. The features may be passed into a several dense layer to yield anatomical structure code. The outputs of the five-layer CNN and the two fully connected layers are then passed through another two fully connected networks (e.g., processed with respect to blocks 1245 and 1247) to provide a final anatomical code decision (e.g., output with respect to block 1249).

The method 1211 includes providing (block 1223) morphological features to a random forest classifier and executing the random forest classifier (block 1225) to output anatomy structure code (block 1227). The morphological features may be derived from the estimation and feature extraction by the sub-algorithm. In this regard, the morphological features may be organized by the evaluation engine according to the left and right WACA rings. For example, because the main "information" for classifying anatomical structure is based on x, y, z position of the ablation points, the evaluation engine executes a sub-algorithm that form rings based on the proximity of ablation points. The evaluation engine extracts from those rings morphological features, for example, a total surface area of the ring, how many points in the ring, and an angle of each point (if the ring is like a "clock"). The morphological features, in an embodiment can be a number along a range of 10 to 50, such as 25.

In accordance with one or more embodiments, the morphological features may be for the nine anatomical structures defined within an algorithm feature space. For instance, the algorithm feature space may include (x_left, y_left, z_left), (x_right, y_right, z_right), where the mean values of all points associated with the left and right rings; (x_norm, y_norm, z_norm), where x, y and z are normalized by range; and (x_norm_ring, y_norm_ring, z_norm_ring), where a normalized point is based on the range of the ring (left or right). The algorithm feature space may further include optional features, such as a VTK file, a minimum distance of ablation points to anatomy, and ablation characteristics (e.g., power, impedance, impedance drop, stability, etc.). The output of the method 1211 may be the anatomical structure code and/or a number within a set of numbers from 1 to 9.

The random forest classifier, in general, includes an ensemble learning method for classification, regression and other tasks that operate by constructing a multitude of decision trees at training time and outputting the class that is the mode of the classes or mean prediction of the individual trees.

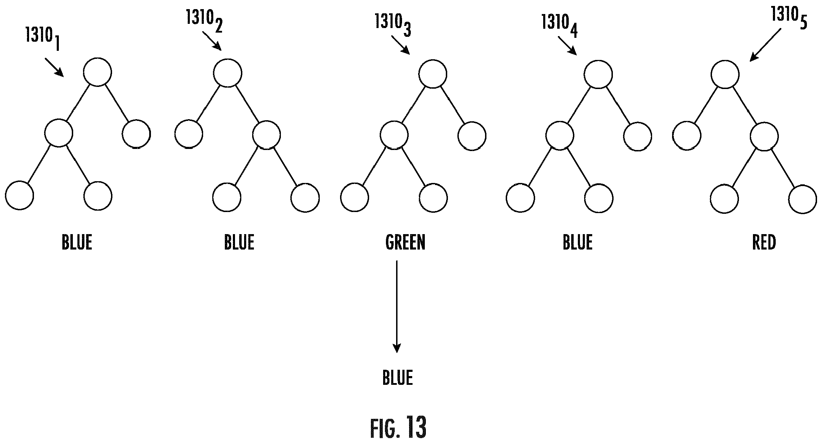

FIG. 13 illustrates an exemplary random forest classifier for classifying the color of a garment is illustrated. As illustrated in FIG. 13, the random forest classifier includes five decision trees 1310₁, 1310₂, 1310₃, 1310₄, and 1310₅ (collectively or generally referred to as decision trees 1310). Each of the trees is designed to classify the color of the garment. In the illustration, three (1310₁, 1310₂, 1310₄) of the five trees determines that the garment is blue, while one determines the garment is green (1310₃) and the remaining tree determines the garment is red (1310₅). The random forest takes these actual predicted values of the five trees and calculates the mode of the actual predicted values to provide random forest answer that the garment is blue.

Returning to FIG. 12, the method 1212 includes leveraging random forest regression, fully connected dense layers, and a CNN architecture. In this way, a five layer CNN receives a VTK anatomy (block 1240), a fully connected layer receives ablation features (block 1241), and a fully connected layer receives morphological features (block 1242). The VTK anatomy may be an anatomy of the left atria stored in the VTK file. IN accordance with one or more embodiments, ablation positions, locations, characteristics, with the VTK file are processed using deep CNN network to obtain set of features that are passed into several dense layers to yield anatomical structure code. The outputs of the five layer CNN and the two fully connected layers are then passed through another two fully connected networks (blocks 1245 and 1247) to provide a final output of anatomy structure codes (block 1249).

Returning to FIG. 7, at block 750, the evaluation engine estimates contiguity of the two separate circular rings around the left and right PVs (e.g., acute reconnection contiguity estimation). Contiguity estimation is an ability by the evaluation engine to determine indirect contact between the two separate circular rings around the left and right PVs and between the two separate circular rings and structural elements outside the left and right pulmonary veins. The output of this estimation can include a probability for contiguity per ablation point. At block 755, the evaluation engine displays the results of estimating contiguity. In this regard, the physician or the medical personnel uses the displayed result for more effective treatment of various cardiovascular diseases

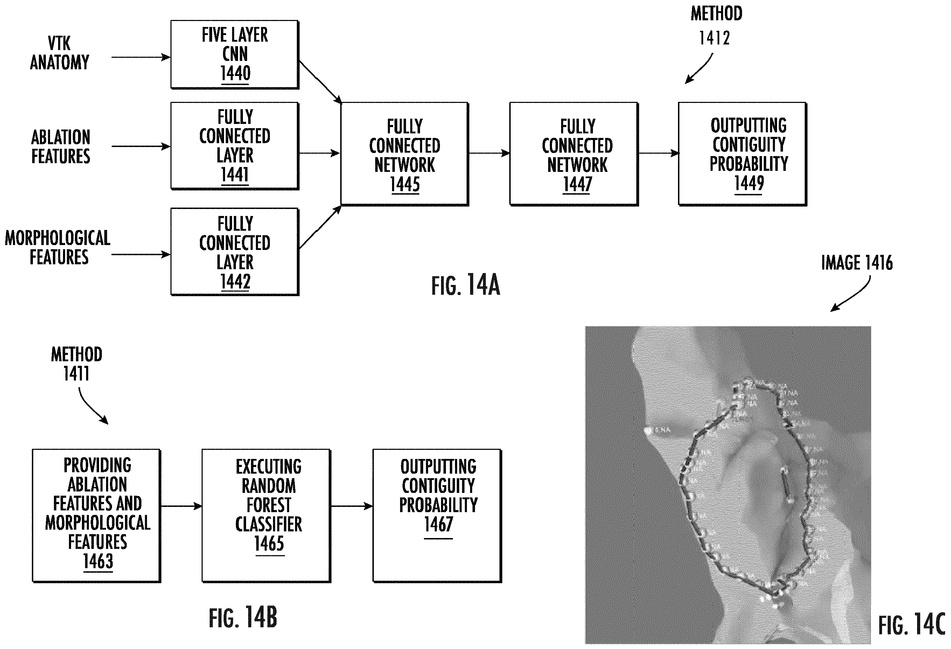

In example operations of block 750, FIG. 14A illustrates a block diagram of classification method 1412, FIG. 14B illustrates a block diagram of classification method 1411, and FIG. 14C illustrates a graphical image 1416. The method 1411 is an example operation of a random forest for acute reconnection classifier (e.g., random forest classifier as described herein). The method 1412 is an example operation of a deep learning method for a redo/acute reconnection classifier. The graphical image 1416 depicts acute reconnection points (denoted in purple).

The method 1411 includes providing (block 1423) ablation features and morphological features to the random forest classifier and executing the random forest classifier (block 1425) to output contiguity probability (block 1427). The ablation features and morphological features for contiguity estimation (redo or acute reconnections) include position based features updated based on the nine anatomical structures defined within an algorithm feature space: (x_left, y_left, z_left), (x_right, y_right, z_right), where the mean values of all points are associated with the left and right rings; (x_norm, y_norm, z_norm), where x, y and z are normalized by range; and (x_norm_ring, y_norm_ring, z_norm_ring), where a normalized point is based on the range of the ring (left or right).

The algorithm feature space may include optional features, such as mesh based features, a VTK file of atrial anatomy (e.g., CNN for feature phase), a minimum distance of ablation points to anatomy, and ablation characteristics (e.g., power, impedance, impedance drop, stability, temperature, RFIndex, FTI, features, time-based features, etc.). The output of the method 1211 may be the anatomical structure code and/or a number within a set of numbers from 1 to 9.

Returning to the method 1412 of FIG. 14A, as discussed herein, the input to the redo/acute reconnection classifier can include a set of characteristics/features per ablation point, a 3D representation of the atria/CT scan/ultrasound/fast anatomical mapping/etc. (e.g., VTK file), and IC ECG or body surface ECG.

The output of the redo/acute reconnection classifier can include a 1 or 0 per voxel of interest, where 1 represents an acute/long-term reconnection site. A voxel can be a point in three-dimensional space, such as a unit of graphic information (e.g., a cube of 2 mm x 2 mm x 2 mm). For instance, as a pixel defines a point in two-dimensional space with x and y coordinates, a voxel requires a third z coordinate. According to one or more embodiments, in 3-D space, each voxel can be further defined in terms of position, color, and density. Based on the output, acute/long-term reconnection sites can be shown.

In accordance with one or more embodiments, the 3D representation of the atria can be processed using the deep CNN network to obtain the set of features, the IC ECG can be processed using deep autoencoder to obtain a set of features. Furthermore, the IC ECG can be processed to obtain additional features, such as bipolar voltage, conduction velocity, cycle length (msec), spatial temporal dispersion level, distance from focal source and fractionation level for each voxel of interest. All features (and characteristics) can be processed by a set of dense neural networks to produce contiguity predictions.

More particularly, the method 1412 includes leveraging random forest classification, fully connected dense layers, and a CNN architecture. According to one or more embodiments, the method 1412 includes leveraging random forest classification for acute/ redo classification and a CNN plus a dense layer for contiguity estimation. In this way, a five-layer CNN receives (block 1440) an anatomy of a left atria from a VTK file, a fully connected layer receives (block 1441) ablation features, and a fully connected layer receives (block 1442) morphological features, along with ablation features in some cases. For example, as described here, ablation positions (x, y, z) locations, and ablation characteristics, are processed with the VTK file using deep CNN network to obtain set of features. Then, all features are passed into a several dense layer to yield anatomical structure code. The outputs of the five-layer CNN and the two fully connected layers are then passed through another two fully connected networks (e.g., processed with respect to blocks 1445 and 1447) to provide a final contiguity probability (e.g., output with respect to block 1449).

At block 760, the evaluation engine generates redo predictions (e.g., predict ablation points annotated as redo). At block 765, the evaluation engine displays the redo predictions results. In this regard, the physician or the medical personnel uses the displayed result for more effective treatment of various cardiovascular diseases.

The flowchart and block diagrams in the figures illustrate the architecture, functionality, and operation of possible implementations of systems, methods, and computer program products according to various embodiments of the present invention. In this regard, each block in the flowchart or block diagrams may represent a module, segment, or portion of instructions, which includes one or more executable instructions for implementing the specified logical function(s). In some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random access memory (RAM), a read-only memory (ROM), an erasable programmable read-only memory (EPROM or Flash memory), a static random access memory (SRAM), and a memory stick. A processor in association with software may be used to implement a radio frequency transceiver for use in a terminal, base station, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof.

The descriptions of the various embodiments herein have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A method comprising:
receiving, by an evaluation engine executed by a processor, a plurality of effective points respective to cardiac tissue of a patient;
determining, by the evaluation engine, an anatomical structural classification for each of the plurality of effective points based on a structural segmentation for the cardiac tissue; and
providing, by the evaluation engine, the anatomical structural classification with the each of the plurality of effective points to support treatment of the cardiac tissue.

2. A system comprising:
a memory configured to store processor executable program instructions of an evaluation engine; and
a processor configured to execute the program instructions of the evaluation engine to cause the apparatus to:
receive a plurality of effective points respective to cardiac tissue of a patient;
determine an anatomical structural classification for each of the plurality of effective points based on a structural segmentation for the cardiac tissue; and
provide the anatomical structural classification with the each of the plurality of effective pointsto support treatment of the cardiac tissue.

3. The method of claim 1 or the system of claim 2, wherein the evaluation engine determines right and left wide area circumferential ablation locations, respective to left and right pulmonary veins of the cardiac tissue, for the plurality of effective points.

4. The method of claim 1 or the system of claim 2, wherein the evaluation engine utilizes manual annotations of training data to determine of the anatomical structural classification.

5. The method of claim 1 or the system of claim 2, wherein the structural segmentation of the cardiac tissue comprises at least one of right posterior, right inferior, right roof, right anterior, left posterior, right inferior, right roof, left ridge, and left anterior of left and right pulmonary veins.

6. The method of claim 1 or the system of claim 2, wherein the structural segmentation of the cardiac tissue comprises at least one of left WACA, right WACA, ostial PVI, roof line, left carina, right carina, posterior line, inferior line, mitral isthmus line, anterior line, anterior line, and cavo-tricuspid isthmus, superior vena cava isolation.

7. The method of claim 1 or the system of claim 2, wherein the evaluation engine extracts morphological features from the plurality of effective points according to the structural segmentation.

8. The method or system of claim 7, wherein an algorithm feature space of the morphological features comprises (x_left, y_left, z_left), (x_right, y_right, z_right); (x_norm, y_norm, z_norm); and (x_norm_ring, y_norm_ring, z_norm_ring).

9. The method of claim 1 or the system of claim 2, wherein the anatomical structural classification comprises an anatomical structure code based on a set of numbers from 1 to 9.

10. The method of claim 1 or the system of claim 2, wherein evaluation engine determines anatomical structure classification based on the effective points and a three-dimensional model of an atria as an input.

11. The method or system of claim 10, wherein evaluation engine determines anatomical structure classification based on at least one of intracardiac electrocardiogram, VTK anatomy file, and body surface ECG.

12. The method or system of claim 11, wherein the VTK anatomy file provide a 3D shell of the atria.
